# EUROPEAN PATENT APPLICATION

(11) **EP 3 111 923 A1**
(43) Date of publication of application: **04.01.2017**
(21) Application number: 16001307.4
(22) Date of filing: 09.06.2016
(51) Int. Cl.: A61K 8/898, A61Q 5/12, C08L 83/08, A61K 8/06

(54) **ORGANOPOLYSILOXANE AND HAIR COSMETIC AND METHOD FOR PRODUCING THEREOF**

(30) Priority: 02.07.2015 JP 2015133216
(71) Applicant: Shin-Etsu Chemical Co., Ltd., Tokyo (JP)
(72) Inventor: Kamei, Masanao, Annaka-shi, Gunma-ken (JP)
(74) Representative: Wibbelmann, Jobst

(57) **Abstract**

The present invention provides an organopolysiloxane having a substituent shown by the following general formula (1),

-R¹-(NR²-R³-)ₙ-NR⁴R⁵ (1)

wherein R¹ represents a divalent hydrocarbon group having 1 to 10 carbon atoms; R², R⁴, and R⁵ each independently represent a hydrogen atom, a monovalent hydrocarbon group having 1 to 10 carbon atoms, a quaternary ammonium salt-containing group shown by the following general formula (2), or a polyoxyalkylene group shown by the following average composition formula (3); R³ represents a divalent hydrocarbon group having 2 to 10 carbon atoms; provided that at least one quaternary ammonium salt-containing group shown by the following general formula (2) and at least one polyoxyalkylene group shown by the following average composition formula (3) are contained in one molecule of the organopolysiloxane; and "n" is 0 to 3; 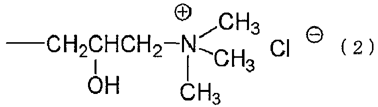 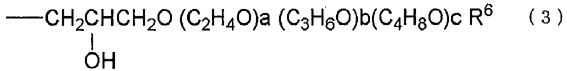 wherein R⁶ represents a hydrogen atom or a monovalent hydrocarbon group having 1 to 30 carbon atoms; "a", "b", and "c" are each independently 0 to 100. This organopolysiloxane can give a hair cosmetic being able to protect hair through the superior adsorptive affinity, to add conditioning effects such as smoothness, easy combing, and softness in a wet condition and in a dry condition, and also able to give gloss in a dry condition.

## Description

### TECHNICAL FIELD

The present invention relates to an organopolysiloxane; a hair cosmetic, and a method for producing thereof.

### BACKGROUND ART

It is known that hair is damaged by perming, bleaching, dyeing, coloring, and so on. Recently, organopolysiloxane has been used as a hair repair or a protective agent to add gloss, smoothness, and softness to this damaged hair. As the organopolysiloxane, although dimethylpolysiloxane ranging over low viscosity one and gummy high polymer can be used, frequently used one is amino-modified organopolysiloxane ranging over low viscosity one and gummy high polymer. It has been known that amino-modified organopolysiloxane, containing an amino group in the molecule, is apt to be adsorbed to hydrophilic moiety of hair and shows superior effect of polysiloxane to give surface protection and softness (PATENT LITERATURE 1, PATENT LITERATURE 2). It has also been presented to use organopolysiloxane having a quaternary ammonium salt-containing group, which improves the adsorptive affinity, in order to improve the persistence of the effect to hair (PATENT LITERATURE 3 to PATENT LITERATURE 7).

It has also been known that having a quaternary ammonium salt-containing group and a polyoxyalkylene group gives easiness of brushing and anti-static property as well as gloss, smoothness, and softness (PATENT LITERATURE 8). In a synthesis of this organopolysiloxane, the N-H bond of the quaternary ammonium salt-containing group and epoxy group-containing polyether are reacted. This reaction, however, having low reactivity, is hard to give the object material with high purity and shows an insufficient effect.

Moreover, it has been proposed many block copolymers in which organopolysiloxane, a quaternary ammonium salt-containing group, and polyoxyalkylene group are contained and bound through various link groups (PATENT LITERATURE 9 to PATENT LITERATURE 15). These block copolymers, however, has a difficulty to control the molecular weight, thereby being unable to give stabilized effect.

### CITATION LIST

### PATENT LITERATURE

PATENT LITERATURE 1: Japanese Patent Laid-Open Publication (Kokai) No. H9-110653
PATENT LITERATURE 2: Japanese Patent Laid-Open Publication (Kokai) No. H1-190619
PATENT LITERATURE 3: Japanese Examined Patent Publication (Kokoku) No. H3-22844
PATENT LITERATURE 4: Japanese Patent No. 2722221
PATENT LITERATURE 5: Japanese Patent No. 2722222
PATENT LITERATURE 6: Japanese Patent No. 3062283
PATENT LITERATURE 7: Japanese Patent No. 3081049
PATENT LITERATURE 8: Japanese Examined Patent Publication (Kokoku) No. H6-96498
PATENT LITERATURE 9: Japanese Patent Laid-Open Publication (Translation of PCT Application) No. 2004-521967
PATENT LITERATURE 10: Japanese Patent No. 4936631
PATENT LITERATURE 11: Japanese Patent No. 4936632
PATENT LITERATURE 12: Japanese Patent No. 4726234
PATENT LITERATURE 13: Japanese Patent No. 4801322
PATENT LITERATURE 14: Japanese Patent Laid-Open Publication (Translation of PCT Application) No. 2006-505644
PATENT LITERATURE 15: Japanese Patent Laid-Open Publication (Translation of PCT Application) No. 2006-505643

### SUMMARY OF INVENTION

### TECHNICAL PROBLEM

The present invention was accomplished in view of the above-described problems. It is an object of the present invention to provide an organopolysiloxane which can give a hair cosmetic being able to protect hair through the superior adsorptive affinity, to add conditioning effects such as smoothness, easy combing, and softness in a wet condition, and to give conditioning effects such as smoothness, easy combing, softness, and gloss even in a dry condition. It is also an object of the present invention to provide a hair cosmetic comprising such organopolysiloxane and a method for producing thereof.

### SOLUTION TO PROBLEM

To solve the foregoing problem, the present invention provides an organopolysiloxane comprising a substituent shown by the following general formula (1),

**-R¹-(NR²-R³-)n-NR⁴R⁵** (1)

wherein R¹ represents a divalent hydrocarbon group having 1 to 10 carbon atoms; R², R⁴, and R⁵ each independently represent a hydrogen atom, a monovalent hydrocarbon group having 1 to 10 carbon atoms, a quaternary ammonium salt-containing group shown by the following general formula (2), or a polyoxyalkylene group shown by the following average composition formula (3); R³ represents a divalent hydrocarbon group having 2 to 10 carbon atoms; provided that at least one quaternary ammonium salt-containing group shown by the following general formula (2) and at least one polyoxyalkylene group shown by the following average composition formula (3) are contained in one molecule of the organopolysiloxane; and "n" is 0 to 3; 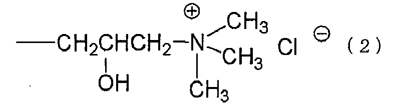 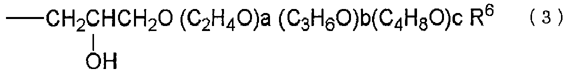 in the formula (3), R⁶ represents a hydrogen atom or a monovalent hydrocarbon group having 1 to 30 carbon atoms; "a", "b", and "c" are each independently 0 to 100.

Such organopolysiloxane can give a hair cosmetic which is able to protect hair through the superior adsorptive affinity, to add conditioning effects such as smoothness, easy combing, and softness in a wet condition, and to give conditioning effects such as smoothness, easy combing, softness, and gloss even in a dry condition.

The organopolysiloxane comprising a substituent shown by the general formula (1) is preferably a compound shown by the following average composition formula (4), 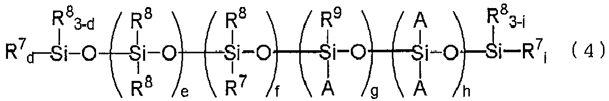 in the formula (4), R⁷ represents a substituent shown by the general formula (1); R⁸ each independently represents a group selected from a hydroxyl group, an alkoxy group having 1 to 3 carbon atoms, an alkyl group having 1 to 30 carbon atoms, a fluoroalkyl group having 1 to 30 carbon atoms, an aryl group having 6 to 30 carbon atoms, an aralkyl group having 7 to 30 carbon atoms, and -R¹-(NH-R³-)ₙNH₂ (wherein R¹, R³, and "n" have the same meanings as defined above); R⁹ is R⁷ or R⁸; and "A" represents an organopolysiloxane segment shown by the following average composition formula (5), 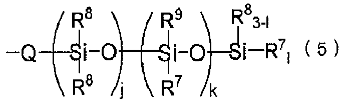
in the average composition formula (5), R⁷, R⁸, and R⁹ have the same meanings as defined above; "Q" represents an oxygen atom or a divalent hydrocarbon group having 1 to 3 carbon atoms;
in the average composition formula (4) and the average composition formula (5), "d", "i", and "l" are each independently 0 to 3; "e" is 0 to 5000, "f" is 0 to 500, "g" is 0 to 100, "h" is 0 to 100, "j" is 0 to 5000, "k" is 0 to 500; provided that when "f" is 0, d+g+i+k+l is 1 or more;
wherein the compound shown by the average composition formula (4) contains at least one R⁷, at least one quaternary ammonium salt-containing group shown by the general formula (2), and at least one polyoxyalkylene group shown by the average composition formula (3).

As the organopolysiloxane comprising a substituent shown by the general formula (1), a compound shown by the average composition formula (4) can be mentioned as described above.

The present invention further provides a hair cosmetic comprising (A) the inventive organopolysiloxane.

Such a hair cosmetic can protect hair through the superior adsorptive affinity, can add conditioning effects such as smoothness, easy combing, and softness in a wet condition, and can give conditioning effects such as smoothness, easy combing, softness, and gloss even in a dry condition.

In this case, it is preferable that the hair cosmetic further contain (B) water and/or water-soluble oil.

Such a hair cosmetic is hard to be gummy form, and accordingly can be treated easily.

In this case, it is preferable that the hair cosmetic further contain (C) oil other than water-soluble oil.

Such a hair cosmetic can further enhance the conditioning effects to hair.

In this case, the hair cosmetic can further contain (D) surfactant selected from cationic surfactant other than the component (A), anionic surfactant, nonionic surfactant, and amphoteric surfactant.

With containing such surfactant, emulsion can be obtained easily.

The water-soluble oil is preferably selected from ethanol, butylene glycol, propylene glycol, glycerin, and mixtures thereof.

Such a hair cosmetic is hard to be gummy form, and accordingly can be treated more easily.

The component (C) is preferably an organopolysiloxane other than the component (A).

In this case, the organopolysiloxane other than the component (A) is a silicone oil selected from cyclic, linear, or branched dimethylpolysiloxane, methylphenylpolysiloxane, amino-modified polysiloxane, and mixtures thereof.

Such a hair cosmetic can further enhance the conditioning effects to hair.

The present invention further provides a method for producing a hair cosmetic, comprising the successive steps of:
preparing an emulsion which contains
   (A) the organopolysiloxane of the present invention,
   (B) water, and
   (C) oil other than water-soluble oil; and adding the emulsion to a hair cosmetic.

Such a method for producing a hair cosmetic can produce a hair cosmetic which is able to protect hair through the superior adsorptive affinity, to add conditioning effects such as smoothness, easy combing, and softness in a wet condition, and to give conditioning effects such as smoothness, easy combing, softness, and gloss even in a dry condition.

In this case, the component (C) is preferably a silicone oil selected from cyclic, linear, or branched dimethylpolysiloxane, methylphenylpolysiloxane, amino-modified polysiloxane, and mixtures thereof.

Such a method for producing a hair cosmetic can produce a hair cosmetic having more enhanced conditioning effects to hair.

In this case, it is preferable that the emulsion further contain (D) surfactant selected from cationic surfactant other than the component (A), anionic surfactant, nonionic surfactant, and amphoteric surfactant.

Such a method for producing a hair cosmetic can easily produce emulsion, and accordingly can easily produce a hair cosmetic which contains the emulsion.

### ADVANTAGEOUS EFFECTS OF INVENTION

The inventive organopolysiloxane can give a hair cosmetic which is able to protect hair through the superior adsorptive affinity, to add conditioning effects such as smoothness, easy combing, and softness in a wet condition, and to give conditioning effects such as smoothness, easy combing, softness, and gloss even in a dry condition. The inventive hair cosmetic can also give these conditioning effects. Moreover, the inventive method for producing a hair cosmetic can produce a hair cosmetic which is able to give these conditioning effects.

### DESCRIPTION OF EMBODIMENTS

Hereinafter, the present invention will be specifically explained.

As described above, it has been desired to provide an organopolysiloxane which can give a hair cosmetic being able to protect hair through the superior adsorptive affinity, to add conditioning effects such as smoothness, easy combing, and softness in a wet condition, and to give conditioning effects such as smoothness, easy combing, softness, and gloss even in a dry condition.

The present inventors diligently study to accomplish the foregoing problems and consequently found that an organopolysiloxane comprising a substituent shown by the following general formula (1) is excellent in adsorptive affinity to hair, particularly to damaged hair, thereby being effective to protect hair: specifically, such organopolysiloxane gives smoothness, easy combing, and softness to hair in a wet condition; gives smoothness, easy combing, softness, and gloss to hair after dried; is excellent in dispersibility and solubility to water with containing the polyoxyalkylene group, and is easily formulated to a hair cosmetic, which is thus improved in stability with time; thereby brought the present invention to completion.

Hereinafter, the embodiments of the present invention will be specifically explained, but the present invention is not limited thereto.

The inventive organopolysiloxane contains a substituent shown by the following general formula (1),

**-R¹-(NR²-R³-)n-NR⁴R⁵** (1)

wherein R¹ represents a divalent hydrocarbon group having 1 to 10 carbon atoms; R², R⁴, and R⁵ each independently represent a hydrogen atom, a monovalent hydrocarbon group having 1 to 10 carbon atoms, a quaternary ammonium salt-containing group shown by the following general formula (2), or a polyoxyalkylene group shown by the following average composition formula (3); R³ represents a divalent hydrocarbon group having 2 to 10 carbon atoms; provided that at least one quaternary ammonium salt-containing group shown by the following general formula (2) and at least one polyoxyalkylene group shown by the following average composition formula (3) are contained in one molecule of the organopolysiloxane; and "n" is 0 to 3; 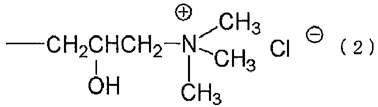 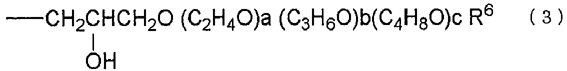 in the formula (3), R⁶ represents a hydrogen atom or a monovalent hydrocarbon group having 1 to 30 carbon atoms; "a", "b", and "c" are each independently 0 to 100.

Such organopolysiloxane can give a hair cosmetic which is able to protect hair through the superior adsorptive affinity, to add conditioning effects such as smoothness, easy combing, and softness in a wet condition, and to give conditioning effects such as smoothness, easy combing, softness, and gloss even in a dry condition.

In the general formula (1), R¹ represents a divalent hydrocarbon group having 1 to 10 carbon atoms, specifically a methylene group, an ethylene group, a propylene group, a butylene group, an isobutylene group, a hexamethylene group, etc., and is preferably a propylene group.

In the general formula (1), R², R⁴, and R⁵ each independently represent a hydrogen atom, a monovalent hydrocarbon group having 1 to 10 carbon atoms, a quaternary ammonium salt-containing group shown by the following general formula (2), or a polyoxyalkylene group shown by the following average composition formula (3), 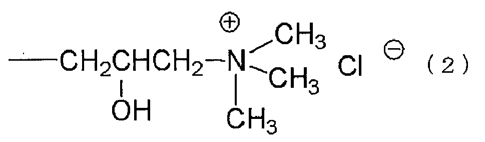 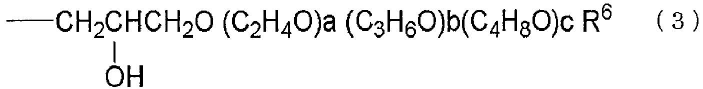 in the formula (3), R⁶, "a", "b", and "c" have the same meanings as defined above.

In the general formula (1), the monovalent hydrocarbon group having 1 to 10 carbon atoms in R², R⁴, and R⁵ each include alkyl groups having 1 to 10 carbon atoms such as a methyl group, an ethyl group, a propyl group, a butyl group, a pentyl group, a hexyl group, a heptyl group, an octyl group, a nonyl group, etc.

In the general formula (1), R², R⁴, and R⁵ are preferably a hydrogen atom, a quaternary ammonium salt-containing group shown by the general formula (2), or a polyoxyalkylene group shown by the average composition formula (3).

In the general formula (1), R³ represents a divalent hydrocarbon group having 2 to 10 carbon atoms, specifically an ethylene group, a propylene group, a butylene group, an isobutylene group, a hexamethylene group, etc., and is preferably an ethylene group.

In the general formula (1), "n" is 0 to 3, preferably 0 or 1. It is to be noted that when "n" is 2 or 3, R² and R³ in each repeating unit may be the same or different from each other.

In the general formula (3), R⁶ represents a hydrogen atom or a monovalent hydrocarbon group having 1 to 30 carbon atoms. Illustrative examples of the monovalent hydrocarbon group having 1 to 30 carbon atoms include a methyl group, an ethyl group, a propyl group, a butyl group, a pentyl group, a decyl group, etc. R⁶ is preferably a hydrogen atom, a methyl group, or a butyl group.

In the general formula (3), "a", "b", and "c" are each independently 0 to 100; preferably, "a" is 0 to 50, "b" is 0 to 50, and "c" is 0 to 20. The polyoxyalkylene group shown by the average composition formula (3) can be either random type or block type.

The inventive organopolysiloxane has a substituent shown by the general formula (1) and contains one or more quaternary ammonium salt-containing group shown by the general formula (2) and one or more polyoxyalkylene group shown by the average composition formula (3) in one molecule.

The inventive organopolysiloxane have only to contain a substituent shown by the general formula (1), and the specific structure is not particularly limited. It is, however, preferably a compound shown by the following average composition formula (4), 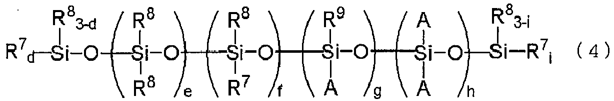 in the formula (4), R⁷ represents a substituent shown by the general formula (1); R⁸ each independently represents a group selected from a hydroxyl group, an alkoxy group having 1 to 3 carbon atoms, an alkyl group having 1 to 30 carbon atoms, a fluoroalkyl group having 1 to 30 carbon atoms, an aryl group having 6 to 30 carbon atoms, an aralkyl group having 7 to 30 carbon atoms, and -R¹-(NH-R³-)ₙNH₂ (wherein R¹, R³, and "n" have the same meanings as defined above); R⁹ is R⁷ or R⁸; and "A" represents an organopolysiloxane segment shown by the following average composition formula (5), 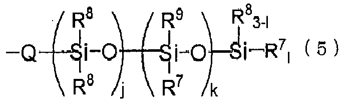
in the average composition formula (5), R⁷, R⁸, and R⁹ have the same meanings as defined above; "Q" represents an oxygen atom or a divalent hydrocarbon group having 1 to 3 carbon atoms;
in the average composition formula (4) and the average composition formula (5), "d", "i", and "l" are each independently 0 to 3; "e" is 0 to 5000, "f" is 0 to 500, "g" is 0 to 100, "h" is 0 to 100, "j" is 0 to 5000, "k" is 0 to 500; provided that when "f" is 0, d+g+i+k+l is 1 or more;
wherein the compound shown by the average composition formula (4) contains at least one R⁷, at least one quaternary ammonium salt-containing group shown by the general formula (2), and at least one polyoxyalkylene group shown by the average composition formula (3).

In the average composition formula (4), R⁸ each independently represents a group selected from a hydroxyl group, an alkoxy group having 1 to 3 carbon atoms, an alkyl group having 1 to 30 carbon atoms, a fluoroalkyl group having 1 to 30 carbon atoms, an aryl group having 6 to 30 carbon atoms, an aralkyl group having 7 to 30 carbon atoms, and -R¹- (NH-R³-)ₙNH₂ (wherein R¹, R³, and "n" have the same meanings as defined above). Illustrative examples of the alkoxy group having 1 to 3 carbon atoms include a methoxy group, an ethoxy group, and a propoxy group. Illustrative examples of the alkyl group having 1 to 30 carbon atoms include alkyl groups such as a methyl group, an ethyl group, a propyl group, a butyl group, a pentyl group, a hexyl group, a heptyl group, an octyl group, a nonyl group, and a decyl group; and cycloalkyl group such as a cyclopentyl group and a cyclohexyl group. Illustrative examples of the fluoroalkyl group having 1 to 30 carbon atoms include a trifluoropropyl group, a heptadecafluorodecyl group, etc. Illustrative examples of the aryl group having 6 to 30 carbon atoms include a phenyl group and a tolyl group. Illustrative examples of the aralkyl group having 7 to 30 carbon atoms include a benzyl group and a phenethyl group. Illustrative examples of the -R¹-(NH-R³-)ₙNH₂ include -C₃H₆-NH₂ and -C₃H₆-NH-C₂H₄-NH₂. R⁸ preferably represents a hydroxyl group, a methyl group, a phenyl group, -C₃H₆-NH₂, or -C₃H₆-NH-C₂H₄-NH₂.

In the average composition formula (5), "Q" represents an oxygen atom or a divalent hydrocarbon group having 1 to 3 carbon atoms. Illustrative examples of the divalent hydrocarbon group having 1 to 3 carbon atoms include a methylene group, an ethylene group, and a propylene group. "Q" preferably represents an ethylene group or a propylene group.

In the average composition formula (4) and the average composition formula (5), "d", "i", and "l" are each independently an integer of 0 to 3, preferably 0 or 1; "e" is 0 to 5000, preferably 10 to 4000; "f" is 0 to 500, preferably 0 to 100; "g" is 0 to 100, preferably 0 to 50; "h" is 0 to 100, preferably 0 to 50; "j" is 0 to 5000, preferably 10 to 4000; "k" is 0 to 500, preferably 0 to 100; provided that when "f" is 0, d+g+i+k+l is 1 or more. The compound shown by the average composition formula (4) contains at least one R⁷, at least one quaternary ammonium salt-containing group shown by the general formula (2), and at least one polyoxyalkylene group shown by the average composition formula (3).

It is to be noted that when the compound shown by the average composition formula (4) has plural of R⁷ (the substituent shown by the general formula (1)), each R⁷ (the kinds of R¹ to R⁵ and the values of "n") may be the same or different from each other.

The inventive organopolysiloxane can be synthesized by the following method.

The inventive organopolysiloxane can be obtained by ring opening reaction of amino group-containing organopolysiloxane, an epoxy group-containing cationization agent shown by the following general formula (6) (glycidyltrimethylammonium chloride), and an epoxy group-containing polyoxyalkylene compound shown by the following average composition formula (7), 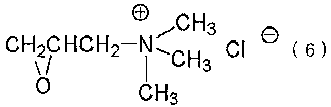 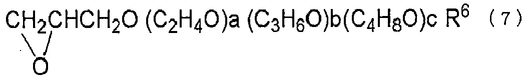 in the formula (7), R⁶, "a", "b", and "c" have the same meanings as defined above.

In the reaction of amino group-containing organopolysiloxane, an epoxy group-containing cationization agent shown by the general formula (6), and an epoxy group-containing polyoxyalkylene compound shown by the average composition formula (7), it is possible to carry out the reaction without a solvent or with using a solvent in accordance with the need. The solvent usable for the reaction is not particularly limited, but illustrative examples thereof include hydrocarbon base solvents such as hexane and toluene; ketone base solvents such as acetone and methyl ethyl ketone; amide base solvents such as N,N-dimethylacetamide, N,N-dimethylformamide, and N-methyl-2-pyrrolidone; ether base solvents such as tetrahydrofuran and 1,4-dioxane; alcohol solvents such as methanol, ethanol, isopropyl alcohol; ester base solvents such as butyl acetate. Among them, ethanol and isopropyl alcohol are particularly preferable.

The reaction temperature is not particularly limited, but preferably 20 to 100°C, more preferably 30 to 60°C. The reaction temperature of 100°C or less can suppress a side reaction such as a decomposition reaction of a cation group, thereby being preferable. The reaction time is not particularly limited, but preferably 1 to 20 hours, more preferably 2 to 5 hours.

The reaction equivalent of the amino group-containing organopolysiloxane and the epoxy group-containing compounds (compounds shown by the general formula (6) and the average composition formula (7)) can be set to 0.1 to 1.1 equivalent, preferably 0.5 to 1.0 equivalent relative to the total amino group. When the amount is 0.1 equivalent or more, it is possible to obtain organopolysiloxane with excellent adhesion property to hair. When the amount is 1.1 equivalent or less, the obtained organopolysiloxane attains a good purity without remaining an unreacted epoxy compound. The equivalent ratio of the epoxy group-containing cationization agent shown by the general formula (6) and the epoxy group-containing polyoxyalkylene compound shown by the average composition formula (7) ((6)/(7)) is not particularly limited. This equivalent ratio ((6)/(7)) can be appropriately selected in accordance with the purpose such as the case intended to dissolve or disperse the obtained organopolysiloxane to water or the case intended to dissolve or disperse the obtained organopolysiloxane to oil solvent. However, the equivalent ratio is preferably in a range of 0.1 to 10.

The present invention further provides a hair cosmetic comprising (A) the inventive organopolysiloxane. Such a hair cosmetic can protect hair through the superior adsorptive affinity, can add conditioning effects such as smoothness, easy combing, and softness in a wet condition, and can give conditioning effects such as smoothness, easy combing, softness, and gloss even in a dry condition.

When using the inventive organopolysiloxane to a hair cosmetic, the content is preferably 0.001 to 20% by mass (hereinafter, it is simply expressed as %), more preferably 0.001 to 10%, further preferably 0.001 to 5% relative to the hair cosmetic in view of obtaining a good conditioning effect and persistence thereof.

To the inventive hair cosmetic, other components to be formulated to a hair cosmetic can be used singly or by appropriately combining two or more kinds. Illustrative examples thereof include the following.

The inventive hair cosmetic preferably contains (B) water and/or water-soluble oil additionally. When the organpolysiloxane of the component (A) is used by dissolving or dispersing to water and/or water-soluble oil as described above, the gummy form can be suppressed compared to the case which contains intact product produced by the foregoing producing method, etc.

Illustrative examples of the water-soluble oil include alcohol base water-soluble oil such as ethanol, butylene glycol, propylene glycol, and glycerin. The water-soluble oil may be used alone or in combination of two or more kinds.

In this case, the hair cosmetic preferably contains (C) oil other than water-soluble oil, additionally. By containing oil other than water-soluble oil of the component (B) as described above, the conditioning effect to hair can be more enhanced.

Illustrative examples of the oil of the component (C) include lower alcohols other than the alcohol base water-soluble oil, saturated or unsaturated alcohols having 12 to 30 carbon atoms; ethers derived from the foregoing alcohol (the alcohol base water-soluble oil and the lower alcohol other than the oil) and polyhydric alcohol; esters derived from the alcohol and fatty acid having 1 to 11 carbon atoms; saturated or unsaturated fatty acid having 12 to 30 carbon atoms; esters derived from the foregoing fatty acid and monohydric or polyhydric alcohol; amides derived from the foregoing fatty acid and amine; sterol; squalene; phospholipids; glycolipid including cationized saccharide; animal oils and fats; vegetable oils and fats; organopolysiloxane other than the component (A). Illustrative examples of this organopolysiloxane other than the component (A) include silicone oils such as cyclic, linear, or branched dimethylpolysiloxane, methylpolysiloxane, amino-modified polysiloxane, alkyl-modified polysiloxane, methylphenylpolysiloxane, polyether-modified silicone. The oil of the component (C) may be used alone or in combination of two or more kinds. The contents of these oil is preferably 0.01 to 30%, more preferably 1 to 25%, further preferably 3 to 20% relative to the hair cosmetic.

In this case, the hair cosmetic can further contain (D) surfactant selected from cationic surfactant other than the component (A), anionic surfactant, nonionic surfactant, and amphoteric surfactant. The surfactant of the component (D) can be any surfactant used in usual hair cosmetics. By containing such a surfactant, emulsion can be easily obtained.

Specific example of the anionic surfactant includes an alkylbenzene sulfonate salt, preferably a linear or a branched alkylbenzene sulfonate salt having an alkyl group having average 10 to 16 carbon atoms; an alkyl ether sulfate salt or an alkenyl ether sulfate salt, preferably an alkyl ether sulfate salt or an alkenyl ether sulfate salt having a linear or a branched alkyl or alkenyl group having average 10 to 20 carbon atoms, wherein the alkyl ether sulfate salt or the alkenyl ether sulfate salt is an adduct of ethylene oxide, propylene oxide, and/or butylene oxide with the average number thereof in one molecule being 0.5 to 8 mole, for example, with molar ratio of ethylene oxide to propylene oxide being 0.1/9.9 to 9.9/0.1 or ethylene oxide to butylene oxide being 0.1/9.9 to 9.9/0.1; an alkylsulfate salt or an alkenylsulfate salt, preferably an alkylsulfate salt or an alkenylsulfate salt having an alkyl group or an alkenyl group having average 10 to 20 carbon atoms; an olefin sulfonate salt, preferably an olefin sulfonate salt having average 10 to 20 carbon atoms in one molecule; an alkane sulfonate salt, preferably an alkane sulfonate salt having average 10 to 20 carbon atoms in one molecule; a higher fatty acid salt, preferably a saturated or an unsaturated fatty acid salt having average 10 to 24 carbon atoms in one molecule; a surfactant of an (amide)ether carboxylic acid type; an α-sulfo fatty acid salt or ester thereof, preferably an α-sulfo fatty acid salt or ester thereof having an alkyl group or an alkenyl group having average 10 to 20 carbon atoms; a surfactant of an N-acyl aminoacid type, preferably a surfactant of an N-acyl aminoacid type having a free carboxylic acid residue and an acyl group having 8 to 24 carbon atoms (for example, N-acyl sarcosinate and N-acyl-β-alanine); a surfactant of a phosphate ester type, preferably a surfactant of a phosphate mono- or diester type having an alkyl group, an alkenyl group, or an alkylene oxide adduct thereof having 8 to 24 carbon atoms; a surfactant of a sulfosuccinate ester type, preferably a sulfosuccinate ester of a higher alcohol having 8 to 22 carbon atoms or an ethoxylate thereof or a sulfosuccinate ester derived from a higher fatty acid amide; a polyoxyalkylene fatty acid amide ether sulfate salt, preferably a sulfate salt of an ethoxylate and so forth of a linear or branched saturated or unsaturated fatty acid monoethanol amide or diethanol amide having 8 to 24 carbon atoms; a monoglyceride sulfate ester salt, preferably a monoglyceride sulfate ester salt having a linear or branched saturated or unsaturated fatty acid group having 8 to 24 carbon atoms; an acylated isethionate salt, preferably an acylated isethionate salt having a linear or branched saturated or unsaturated fatty acid group having 8 to 24 carbon atoms; an alkyl glyceryl ether sulfate salt or an alkyl glyceryl ether sulfonate salt, preferably an alkyl glyceryl ether sulfate salt or an alkyl glyceryl ether sulfonate salt having a linear or branched alkyl group, alkenyl group, or alkylene oxide adduct thereof having 8 to 24 carbon atoms; an alkyl or alkenyl amide sulfonate, preferably an alkyl or alkenyl amide sulfonate having a linear or branched alkyl or alkenyl group having 8 to 24 carbon atoms; an alkanol amide sulfosuccinate salt, preferably an alkanol amide sulfosuccinate salt having a linear or branched alkyl or alkenyl group having 8 to 24 carbon atoms; an alkyl sulfoacetate, preferably an alkyl sulfoacetate having a linear or a branched alkyl or alkenyl group having 8 to 24 carbon atoms; an acylated taurate, preferably an acylated taurate having a linear or branched saturated or unsaturated fatty acid group having 8 to 24 carbon atoms; an N-acyl-N-carboxyethyl glycine salt, preferably an N-acyl-N-carboxyethyl glycine salt having an acyl group having 6 to 24 carbon atoms.

Illustrative examples of the salt of the anionic surfactant, that is the counter ion of an anionic residue thereof includes an ion of alkali metal such as sodium and potassium; an ion of alkaline earth metal such as calcium and magnesium; an ammonium ion, and an alkanolamine having 1 to 3 alkanol groups with 2 or 3 carbon atoms (such as monoethanol amine, diethanol amine, triethanol amine, and triisopropanol amine).

Preferable example of the above anionic surfactant includes an alkyl ether sulfate salt, and particularly a polyoxyethylene alkyl ether sulfate salt.

Illustrative examples of the nonionic surfactant include a polyoxyalkylene alkyl ether or a polyoxyalkylene alkenyl ether having a linear or branched alkyl or alkenyl group having average 10 to 24 carbon atoms and having an adduct of ethylene oxide, propylene oxide, or butylene oxide; a glycerin ester of a fatty acid having 8 to 20 carbon atoms; a glycol ester of a fatty acid having 8 to 20 carbon atoms; an alkylene oxide adduct of monoglyceride of a fatty acid having 8 to 20 carbon atoms; a sucrose ester of a fatty acid having 8 to 20 carbon atoms; a sorbitan ester of a fatty acid having 8 to 20 carbon atoms; a polyglycerin fatty acid ester having an acyl group having 8 to 20 carbon atoms; a monoethanol amide, diethanol amide, or their ethoxylate of a fatty acid having 8 to 20 carbon atoms; a polyoxyethylene hard castor oil; a polyoxyalkylene sorbitol fatty acid ester having an acyl group having 8 to 20 carbon atoms; a polyoxyethylene sorbit fatty acid ester having an acyl group having 8 to 20 carbon atoms; an alkyl saccharide surfactant having a linear or branched alkyl group, an alkenyl group, or an alkyl phenyl group having 8 to 18 carbon atoms; an alkylamine oxide or an alkylamide amine oxide having a linear or branched alkyl group or an alkenyl group having 8 to 20 carbon atoms; an ether compound or an ester compound of a polyhydric alcohol having a linear or branched alkyl group or an alkenyl group having 8 to 20 carbon atoms; an organopolysiloxane modified with polyoxyalkylene, an organopolysiloxane co-modified with polyoxyalkylene and an alkyl, an organopolysiloxane modified with polyglycerin, an organopolysiloxane co-modified with polyglycerin and an alkyl, an organopolysiloxane co-modified with a polyoxyalkylene and a fluoroalkyl, a crosslinking polyoxyalkylene organopolysiloxane, a sugar-modified silicone, an oxazoline-modified silicone; a polyoxyalkylene alkyl aryl ether, a polyoxyalkylene lanolin alcohol, a polyoxyalkylene fatty acid ester; a pluronic block polymer, a tetronic block polymer; a polyoxyalkylene fatty acid amide, a polyoxyalkylene alkylamide, and a polyethylene imine derivative.

As to the amphoteric surfactant, there is no particular restriction provided that it is used in a usual hair cosmetic; and illustrative examples thereof include an amide aminoacid type, a carbobetaine type, an amide betaine type, a sulfobetaine type, an amide sulfobetaine type, an imidazolinium betaine type, an aminoacid type, a phosphobetaine type, and a phosphate ester type.

As to the cationic surfactant, a tertiary amine, a quaternary ammonium salt, an amide amine, an ester amine, and so forth may be mentioned. Illustrative examples thereof include behenyl trimethyl ammonium chloride, distearyl dimethyl ammonium chloride, cetyl trimethyl ammonium chloride, stearyl trimethyl ammonium chloride, lauryl trimethyl ammonium chloride, N-stearyl-N,N,N-tri(polyoxyethylene) ammonium chloride (added with total 3 ethylene oxides), cetyl benzyl dimethyl ammonium chloride, cetyl triethyl ammonium bromide, distearyl dimethyl ammonium chloride, 2-decyltetradecyl trimethyl ammonium chloride, 2-dodecylhexadecyl trimethyl-ammonium chloride, di-2-hexyldecyl dimethyl ammonium chloride, di-2-octyldodecyl dimethyl ammonium chloride, behenyl tertiary amine, stearyl tertiary amine, and stearamidepropyl dimethyl amine.

The content of the surfactant is preferably 0.1 to 50%, more preferably 0.5 to 40% relative to the hair cosmetic, and further preferably 1 to 30% in view of foaming property.

The inventive hair cosmetic can be further formulated a thickener such as hydroxyethyl cellulose; an anionic, amphoteric, cationic, and nonionic polymer; a fragrance, a pearlescent aid, a hair setting polymer, a pigment, an UV absorber, an antioxidant, a preservative, etc. in accordance with the need.

When the hair cosmetic of the present invention is in a form of hair setting, hair foaming, hair spray, and so forth, it is preferable to contain following exemplified compounds as a hair setting polymer therein.

Illustrative examples of the compound include polyvinyl pyrrolidone; copolymer of polyvinyl pyrrolidone and vinyl acetate; crosslinked copolymer of polyvinyl pyrrolidone, vinyl acetate, and vinyl propionate; copolymer of polyvinyl pyrrolidone and an alkyl aminoacrylate; copolymer of polyvinyl pyrrolidone, an acrylate, and (meth)acrylic acid; copolymer of polyvinyl pyrrolidone, an alkyl aminoacrylate, and vinyl caprolactam; copolymer of methyl vinyl ether and a maleic anhydride alkyl half ester; copolymer of vinyl acetate and crotonic acid; copolymer of vinyl acetate, crotonic acid, and vinyl neodecanoate; copolymer of vinyl acetate, crotonic acid, and vinyl propionate; copolymer of vinyl acetate, vinyl tert-butylbenzoate, and crotonic acid; copolymer of (meth)acrylic acid and a (meth)acrylate ester; copolymer of acrylic acid, an alkyl acrylate ester, and an alkyl acrylamide; copolymer of (meth)acryl ethyl betaine and an alkyl (meth)acrylate ester; copolymer of N-methacryloyloxyethyl-N,N-dimethyl ammonium α-N-methylcarboxy betaine and an alkyl (meth)acrylate ester; copolymer of an alkyl acrylate ester, butylaminoethyl methacrylate, and acrylic acid octylamide; a basic acryl polymer; silicone compounds such as an acrylic copolymer of a radical polymerizable monomer and a silicone macromer having a radical polymerizable group at one terminal, trimethyl silicate; a compound having a cellulose skeleton, a cationic cellulose derivative; a salt of hydroxypropyl chitosan, carboxymethyl chitin, carboxymethyl chitosan, and chitosan with a monovalent acid such as pyrrolidone carboxylic acid, lactic acid, and glycol acid or with a divalent acid such as adipic acid and succinic acid; and a water-dispersible polyester.

The content of the hair setting polymer in the hair cosmetic is preferably 0.1 to 10%, more preferably 0.5 to 6%, and further preferably 1 to 4% in order to obtain necessary and sufficient hair setting force.

The producing method of the inventive hair cosmetic is not particularly limited. For example, each components such as the components (A) to (D) can be separately added to a hair cosmetic.

It is also possible to prepare an emulsion which contains (A) the inventive organopolysiloxane, (B) water, and (C) oil other than water-soluble oil; and to add the emulsion to a hair cosmetic subsequently. In this case, the foregoing silicone oil is preferably used as the component (C). In this case, it is also possible to add the foregoing surfactant of the component (D) when preparing the emulsion to prepare an emulsion which contains the components (A) to (D). Moreover, it is also possible to previously prepare a solution in which the component (A) is dissolved or dispersed to water and/or water-soluble oil, and then to prepare the emulsion from the solution and the components (B) to (C).

These producing method of a hair cosmetic can produce a hair cosmetic which is able to protect hair through the superior adsorptive affinity, to add conditioning effects such as smoothness, easy combing, and softness in a wet condition, and to give conditioning effects such as smoothness, easy combing, softness, and gloss even in a dry condition. Furthermore, the hair cosmetic obtained by the method to prepare an emulsion can be suitably used as a hair treatment with faint acidity.

Illustrative examples of the inventive hair cosmetic include a hair shampoo, a hair rinse, a hair treatment, and a hair conditioner used in the bathroom; a hair form, a hair spray, a hair cream, a hair wax, and a hair gel used outside the bathroom; and an agent used at home or a beauty salon for beauty treatment such as a hair dye, a permanent wave agent, a hair manicure, and a hair bleach. The inventive organopolysiloxane can be formulated into any of these agents. The present invention is suitable for a hair treatment, a hair conditioner, and so on, because of the superior effect for protecting hair; and particularly suitable for damaged hair because of the remarkable effect to hair damaged by bleach and so on.

### EXAMPLES

Hereinafter, the present invention will be explained more specifically by showing Synthesis Examples, Examples, and Comparative Examples, but the present invention is not limited thereto. It is to be noted that the amount of each component in the Table is an amount in terms of the pure material unless otherwise noted.

### [Synthesis Example 1]

Into a reactor, 65 parts by mass of amino-modified organopolysiloxane with an amine equivalent of 650 g/mol shown by the following average composition formula (8), 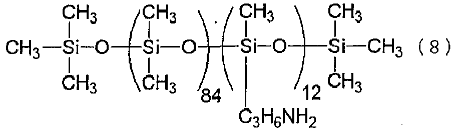 50 parts by mass of isopropyl alcohol, 9.5 parts by mass of 80wt% aqueous solution of an epoxy group-containing cationization agent shown by the following general formula (6), and 67.5 parts by mass of an epoxy group-containing polyoxyalkylene compound shown by the following general formula (9) were added under a nitrogen atmosphere, and stirred at 50°C for 3 hours. 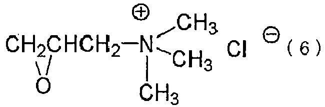 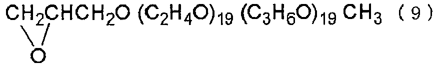 The obtained reaction mixture was stripped at 60°C under reduced pressure to remove the solvent, and to give 134.1 g (yield: 95.6%) of yellow transparent gummy organopolysiloxane shown by the following average composition formula (10).

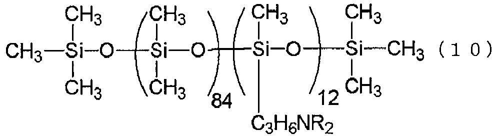

In the average composition formula (10), "R" was contained in an average molar ratio of the following formula (2)/the following formula (11)/H = 6/4/14.

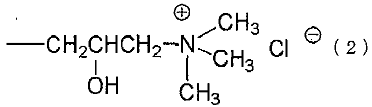

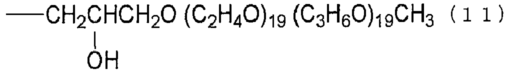

### [Synthesis Example 2]

Into a reactor, 32 parts by mass of amino-modified organopolysiloxane with an amine equivalent of 320 g/mol shown by the following average composition formula (12), 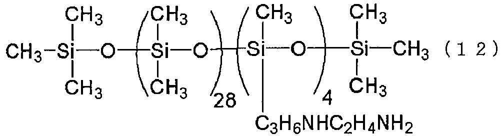 50 parts by mass of isopropyl alcohol, 9.4 parts by mass of 80wt% aqueous solution of an epoxy group-containing cationization agent shown by the following general formula (6), and 48.3 parts by mass of an epoxy group-containing polyoxyalkylene compound shown by the following general formula (13) were added under a nitrogen atmosphere, and stirred at 50°C for 3 hours. 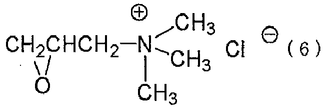 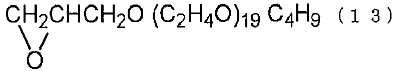 The obtained reaction mixture was stripped at 60°C under reduced pressure to remove the solvent, and to give 82.9 g (yield: 94.5%) of yellow transparent gummy organopolysiloxane shown by the following average composition formula (14).

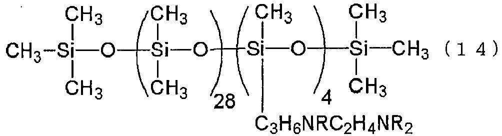

In the average composition formula (14), "R" was contained in an average molar ratio of the following formula (2)/the following formula (15)/H = 4/4/4.

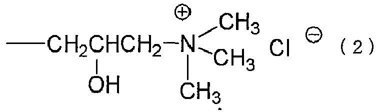

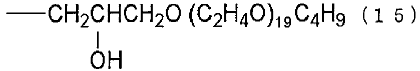

### [Synthesis Example 3]

Into a reactor, 56.4 parts by mass of amino-modified organopolysiloxane with an amine equivalent of 5640 g/mol shown by the following average composition formula (16), 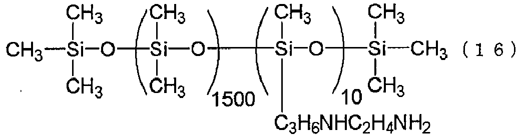 50 parts by mass of isopropyl alcohol, 1.9 parts by mass of 80wt% aqueous solution of an epoxy group-containing cationization agent shown by the following general formula (6), and 7 parts by mass of an epoxy group-containing polyoxyalkylene compound shown by the following general formula (17) were added under a nitrogen atmosphere, and stirred at 50°C for 3 hours. 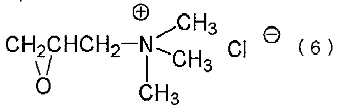 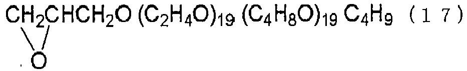 The obtained reaction mixture was stripped at 60°C under reduced pressure to remove the solvent, and to give 61.0 g (yield: 93.3%) of yellow transparent gummy organopolysiloxane shown by the following average composition formula (18).

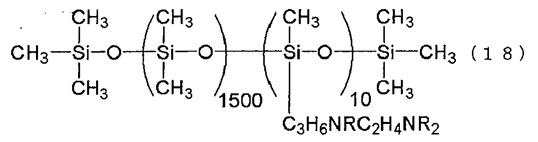

In the average composition formula (18), "R" was contained in an average molar ratio of the following formula (2)/the following formula (19)/H = 7/3/10.

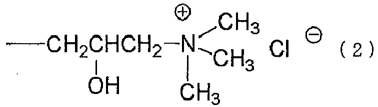

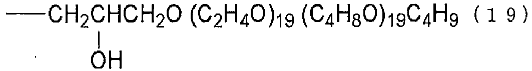

### [Synthesis Example 4]

Into a reactor, 60 parts by mass of amino-modified organopolysiloxane with an amine equivalent of 1200 g/mol shown by the following average composition formula (20), 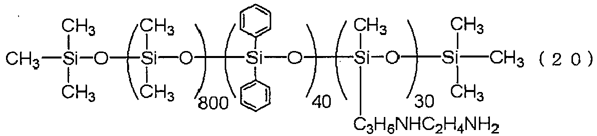 50 parts by mass of isopropyl alcohol, 4.7 parts by mass of 80wt% aqueous solution of an epoxy group-containing cationization agent shown by the following general formula (6), and 1.9 parts by mass of glycidol were added under a nitrogen atmosphere, and stirred at 50°C for 3 hours. 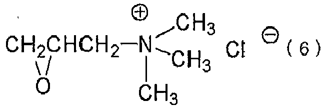 The obtained reaction mixture was stripped at 60°C under reduced pressure to remove the solvent, and to give 61.0 g (yield: 92.9%) of yellow transparent gummy organopolysiloxane shown by the following average composition formula (21).

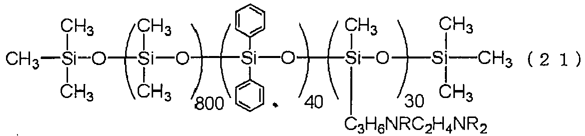

In the average composition formula (21), "R" was contained in an average molar ratio of the following formula (2)/the following formula (22)/H = 15/15/30.

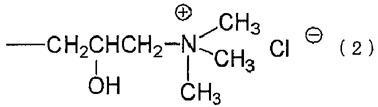

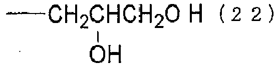

### [Synthesis Example 5]

Into a reactor, 39 parts by mass of amino-modified organopolysiloxane with an amine equivalent of 390 g/mol shown by the following average composition formula (23), 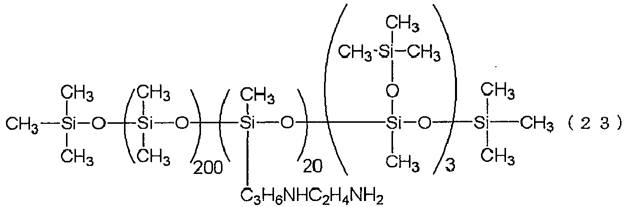 50 parts by mass of isopropyl alcohol, 7.1 parts by mass of 80wt% aqueous solution of an epoxy group-containing cationization agent shown by the following general formula (6), and 31.9 parts by mass of an epoxy group-containing polyoxyalkylene compound shown by the following general formula (24) were added under a nitrogen atmosphere, and stirred at 50°C for 3 hours. 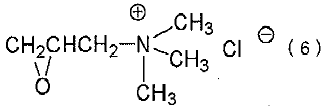 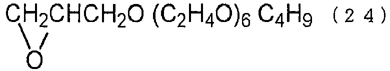 The obtained reaction mixture was stripped at 60°C under reduced pressure to remove the solvent, and to give 73.6 g (yield: 96.1%.) of yellow transparent gummy organopolysiloxane shown by the following average composition formula (25).

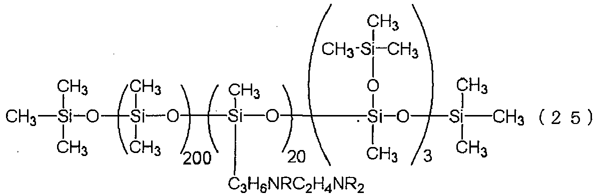

In the average composition formula (25), "R" was contained in an average molar ratio of the following formula (2)/the following formula (26)/H = 1/1/1.

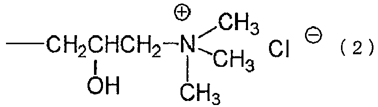

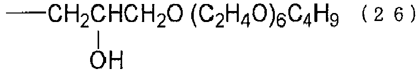

### [Comparative Synthesis Example 1]

Into a reactor, 65 parts by mass of amino-modified organopolysiloxane with an amine equivalent of 650 g/mol shown by the following average composition formula (8), 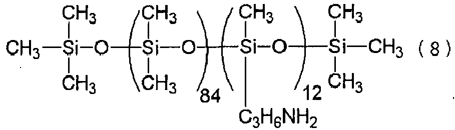 50 parts by mass of isopropyl alcohol, and 9.5 parts by mass of 80wt% aqueous solution of an epoxy group-containing cationization agent shown by the following general formula (6) were added under a nitrogen atmosphere, and stirred at 50°C for 3 hours. 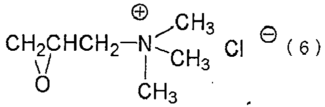 The obtained reaction mixture was stripped at 60°C under reduced pressure to remove the solvent, and to give 68.6 g (yield: 94.5%) of yellow transparent gummy organopolysiloxane shown by the following average composition formula (27).

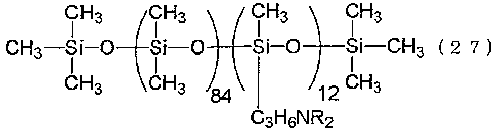

In the average composition formula (27), "R" was contained in an average molar ratio of the following formula (2)/ H = 6/18.

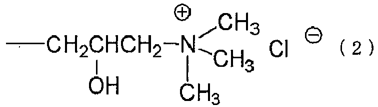

### [Comparative Synthesis Example 2]

Into a reactor, 65 parts by mass of amino-modified organopolysiloxane with an amine equivalent of 650 g/mol shown by the following average composition formula (8), 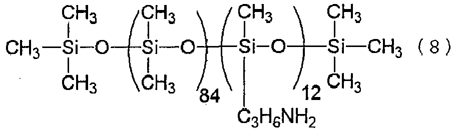 50 parts by mass of isopropyl alcohol, 67.5 parts by mass of an epoxy group-containing polyoxyalkylene compound shown by the following general formula (9) were added under a nitrogen atmosphere, and stirred at 50°C for 3 hours. 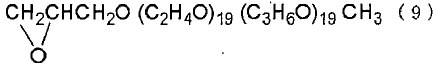 The obtained reaction mixture was stripped at 60°C under reduced pressure to remove the solvent, and to give 127.2 g (yield: 96.0%) of yellow transparent liquid organopolysiloxane shown by the following average composition formula (28).

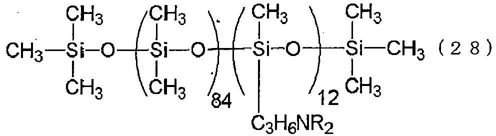

In the average composition formula (28), "R" was contained in an average molar ratio of the following formula (11)/H = 10/14.

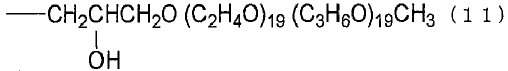

### [Examples 1 to 5 and Comparative Examples 1 to 3]

By using the organopolysiloxanes prepared in the foregoing Synthesis Examples 1 to 5 and Comparative Synthesis Examples 1 and 2, and commercially available amino-modified silicone, hair conditioners of the compositions shown in the following Table 1 were prepared by a conventional method.

On the obtained hair conditioners, organoleptic evaluations were carried out by the following methods. The results are shown in the following Table 1.

### [Organoleptic evaluation methods]

By using hair bunches (each 20 g, 20 cm) of Japanese women subjected to a bleach treatment with a commercially available bleach agent, the organoleptic evaluations were carried out by 5 members of panelists through -treatments according to the following method. To a hair bunch washed with 3 g of the standard shampoo having the following composition, 2 g of a hair conditioner shown in Table 1 was applied and sufficiently fitted to the entire hair. Then, the evaluations in a wet condition were carried out under running water at about 40°C for about 30 seconds. Subsequently, they were subjected to towel drying, dried with a dryer, and then the evaluations in a dry condition were carried out. The organoleptic evaluations were carried out on smoothness, easy combing, and softness of the hair in a wet condition; and smoothness, easy combing, and softness of the hair, and existence of gloss-addition effect in a dry condition. The results are indicated with the following evaluation criteria on the basis of numbers of panelists answered as "effective."

The formulation of the standard shampoo:

| | |
|---|---|
| 25% sodium salt of polyoxyethylene(2.5) laurylethersulfate | 62.0% |
| lauric diethanolamide | 2.3% |
| disodium EDTA | 0.15% |
| sodium benzoate | 0.5% |
| sodium chloride | 0.8% |
| 75% phosphoric acid | appropriate |
| fragrance | appropriate |
| methyl paraben | appropriate |
| purified water | remainder |
| total | 100.0% |

### [Evaluation criteria]

Ideal: 4 to 5 panelists answered as "effective"
Good: 3 panelists answered as "effective"
Fair: 2 panelists answered as "effective" Poor: 1 or no panelist answered as "effective"

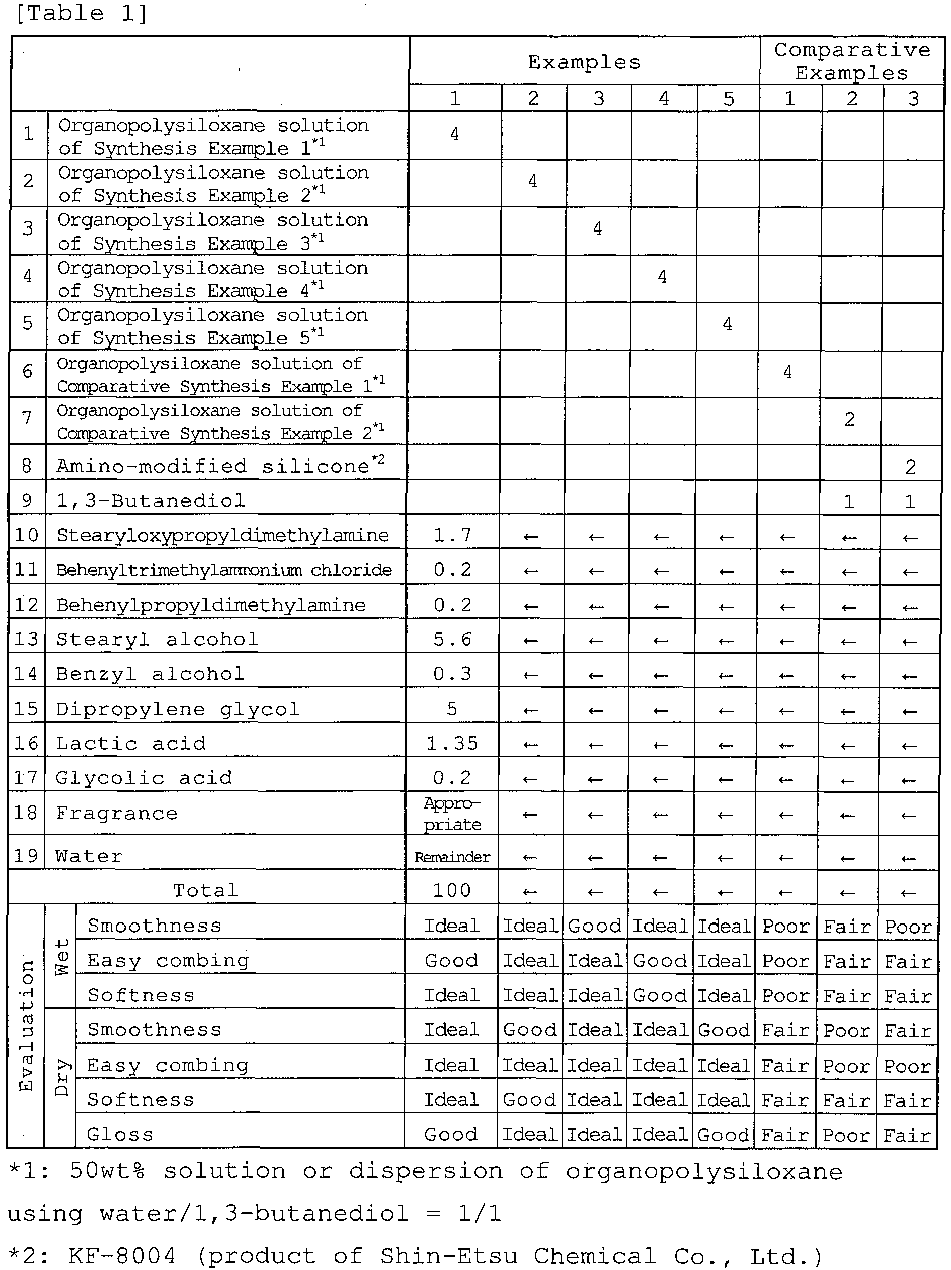

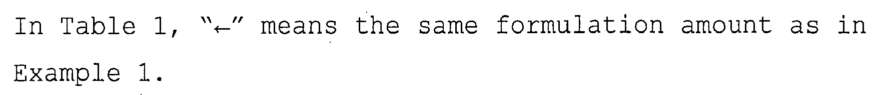

As shown in Table 1, the hair conditioners which contained inventive organopolysiloxane (Examples 1 to 5) gave favorable results of smoothness, easy combing, and softness in the wet condition; and also gave favorable results of smoothness, easy combing, softness, and gloss in the dry condition. On the other hand, the hair conditioner which contained organopolysiloxane without a polyoxyalkylene group (Comparative Example 1) gave poor results particularly on the smoothness, easy combing, and softness in the wet condition. The hair conditioner which contained organopolysiloxane without a quaternary ammonium salt-containing group (Comparative Example 2) gave poor results particularly on the smoothness, easy combing, and gloss in the dry condition. The hair conditioner which contains commercially available amino-modified silicone instead of the inventive organopolysiloxane (Comparative Example 3) gave poor results in both of the wet condition and the dry condition.

### [Example 6]

The hair treatment with the following composition (pH 7.0) was prepared by a conventional method:

| | |
|---|---|
| octadecyloxy(2-hydroxypropyl)dimethylamine | 0.5% |
| stearic dimethylaminopropylamide | 2.0% |
| stearyl alcohol | 5.0% |
| dipropylene glycol | 1.0% |
| benzyl alcohol | 0.5% |
| phenoxy ethanol | 0.1% |
| organopolysiloxane solution obtained by Synthesis Example 1*¹ | 5.0% |
| highly polymerized dimethylpolysiloxane^{*3} | 0.5% |
| glycerin | 5.0% |
| polypropylene glycol | 2.5% |
| lanolin fatty acid | 0.5% |
| sunflower oil | 0.5% |
| lactic acid | 1.5% |
| fragrance | 0.4% |
| ion-exchange water | remainder |
| total | 100.0% |

| | |
|---|---|
| *1: 50wt% solution or dispersion of organopolysiloxane using water/1,3-butanediol = 1/1 *3: KF-96H 100000cs (product of Shin-Etsu Chemical Co., Ltd.) | |

The obtained hair treatment gave favorable results of smoothness, softness, and flatness in rinsing; and favorable results of smoothness, softness, and easy combing after drying.

### [Example 7]

The hair treatment with the following composition (pH 4.0) was prepared by a conventional method

| | |
|---|---|
| dimethyl behenamine | 2.0% |
| behenyltrimethylammonium chloride | 0.3% |
| stearyl alcohol | 4.5% |
| behenyl alcohol | 1.5% |
| isononyl isononanoate | 0.5% |
| emulsion which contains organopolysiloxane obtained by Synthesis Example 2^{*4} | 5.0% |
| amino-modified organopolysiloxane^{*5} | 0.5% |
| glycolic acid | 0.5% |
| malic acid | 0.1% |
| dipropylene glycol | 3.0% |
| benzyl alcohol | 0.3% |
| arginine | 0.2% |
| pantothenyl ethyl ether | 0.1% |
| hydrolyzed conchiolin liquid (dry portion 3%) | 0.05% |
| panax ginseng extract (dry portion 3%) | 0.05% |
| soybean extract (dry portion 0.4%) | 0.05% |
| eucalyptus extract (dry portion 0.2%) | 0.05% |
| rice germ oil | 0.05% |
| fragrance | appropriate |
| methyl paraben | appropriate |
| purified water | remainder |
| total | 100.0% |

| | |
|---|---|
| *4: emulsion obtained from 70 parts by mass of solution of dimethylpolysiloxane with polymerization degree of 3,000/ KF-96A 20cs (product of Shin-Etsu Chemical Co., Ltd.) = 20/80, 10 parts by mass of 50wt% water/1,3-butanediol =1/1 solution of organopolysiloxane of Synthesis Example 2, and 20 parts by mass of water *5: KF-8004 (product of Shin-Etsu Chemical Co., Ltd.) | |

The obtained hair treatment gave favorable results of smoothness, softness, and flatness in rinsing; and favorable results of smoothness, softness, and easy combing after drying.

It is to be noted that the present invention is not restricted to the foregoing embodiment. The embodiment is just an exemplification, and any examples that have substantially the same feature and demonstrate the same functions and effects as those in the technical concept described in claims of the present invention are included in the technical scope of the present invention.

## Claims

1. An organopolysiloxane comprising a substituent shown by the following general formula (1),
**-R¹-(NR²-R³-)n-NR⁴R⁵** (1)
wherein R¹ represents a divalent hydrocarbon group having 1 to 10 carbon atoms; R², R⁴, and R⁵ each independently represent a hydrogen atom, a monovalent hydrocarbon group having 1 to 10 carbon atoms, a quaternary ammonium salt-containing group shown by the following general formula (2), or a polyoxyalkylene group shown by the following average composition formula (3); R³ represents a divalent hydrocarbon group having 2 to 10 carbon atoms; provided that at least one quaternary ammonium salt-containing group shown by the following general formula (2) and at least one polyoxyalkylene group shown by the following average composition formula (3) are contained in one molecule of the organopolysiloxane; and "n" is 0 to 3; 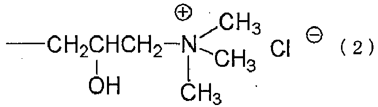 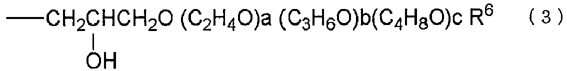 in the formula (3), R⁶ represents a hydrogen atom or a monovalent hydrocarbon group having 1 to 30 carbon atoms; "a", "b", and "c" are each independently 0 to 100.

2. The organopolysiloxane according to claim 1, wherein the organopolysiloxane comprising a substituent shown by the general formula (1) is a compound shown by the following average composition formula (4), 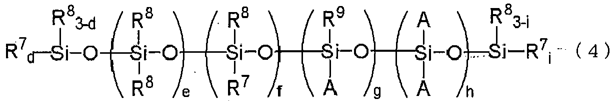 in the formula (4), R⁷ represents a substituent shown by the general formula (1); R⁸ each independently represents a group selected from a hydroxyl group, an alkoxy group having 1 to 3 carbon atoms, an alkyl group having 1 to 30 carbon atoms, a fluoroalkyl group having 1 to 30 carbon atoms, an aryl group having 6 to 30 carbon atoms, an aralkyl group having 7 to 30 carbon atoms, and -R¹-(NH-R³-)ₙNH₂ (wherein R¹, R³, and "n" have the same meanings as defined above); R⁹ is R⁷ or R⁸; and "A" represents an organopolysiloxane segment shown by the following average composition formula (5), 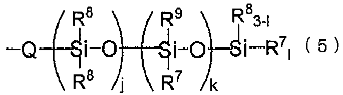
in the average composition formula (5), R⁷, R⁸, and R⁹ have the same meanings as defined above; "Q" represents an oxygen atom or a divalent hydrocarbon group having 1 to 3 carbon atoms;
in the average composition formula (4) and the average composition formula (5), "d", "i", and "l" are each independently 0 to 3; "e" is 0 to 5000, "f" is 0 to 500, "g" is 0 to 100, "h" is 0 to 100, "j" is 0 to 5000, "k" is 0 to 500; provided that when "f" is 0, d+g+i+k+l is 1 or more;
wherein the compound shown by the average composition formula (4) contains at least one R⁷, at least one quaternary ammonium salt-containing group shown by the general formula (2), and at least one polyoxyalkylene group shown by the average composition formula (3).

3. A hair cosmetic comprising (A) the organopolysiloxane according to claim 1 or 2.

4. The hair cosmetic according to claim 3, further comprising (B) water and/or water-soluble oil.

5. The hair cosmetic according to claim 3 or 4, further comprising (C) oil other than water-soluble oil.

6. The hair cosmetic according to any one of claims 3 to 5, further comprising (D) surfactant selected from cationic surfactant other than the component (A), anionic surfactant, nonionic surfactant, and amphoteric surfactant.

7. The hair cosmetic according to any one of claims 4 to 6, wherein the water-soluble oil is selected from ethanol, butylene glycol, propylene glycol, glycerin, and mixtures thereof.

8. The hair cosmetic according to any one of claims 5 to 7, wherein the component (C) is an organopolysiloxane other than the component (A).

9. The hair cosmetic according to claim 8, wherein the organopolysiloxane other than the component (A) is a silicone oil selected from cyclic, linear, or branched dimethylpolysiloxane, methylphenylpolysiloxane, amino-modified polysiloxane, and mixtures thereof.

10. A method for producing a hair cosmetic, comprising the successive steps of:
preparing an emulsion which contains
(A) the organopolysiloxane according to claim 1 or 2,
(B) water, and
(C) oil other than water-soluble oil; and adding the emulsion to a hair cosmetic.

11. The method for producing a hair cosmetic according to claim 10, wherein the component (C) is a silicone oil selected from cyclic, linear, or branched dimethylpolysiloxane, methylphenylpolysiloxane, amino-modified polysiloxane, and mixtures thereof.

12. The method for producing a hair cosmetic according to claim 10 or 11, wherein the emulsion further contains (D) surfactant selected from a cationic surfactant other than the component (A), anionic surfactant, nonionic surfactant, and amphoteric surfactant.
